# FASCICULE DE BREVET EUROPEEN

(11) **EP 0 587 642 B1**
(45) Date de publication et mention de la délivrance du brevet: **19.04.1995**
(21) Numéro de dépôt: 92911149.0
(22) Date de dépôt: 21.05.1992
(51) Int. Cl.: A61F 11/00

(54) **DISPOSITIF NOTAMMENT DESTINE A L'HYGIENE DES CONDUITS AUDITIFS DE PERSONNES**
VORRICHTUNG ZUR REINIGUNG VON GEHOERGAENGEN DER MENSCHLICHEN OHREN
DEVICE PARTICULARLY INTENDED FOR THE HYGIENE OF THE AUDITORY MEATUS

(30) Priorité: 31.05.1991 FR 9106835
(43) Date de publication de la demande: 23.03.1994
(73) Titulaire: SARL "P.N.B.", 59200 Tourcoing (FR)
(72) Inventeur: Vanraes, Pierre, F-59200 Tourcoing (FR)
(74) Mandataire: Ecrepont, Robert
(86) Numéro de dépôt international: FR9200456
(87) Numéro de publication internationale: WO9221308

(56) Documents cités:
- FR-A- 2 277 557
- FR-A- 2 551 657
- US-A- 1 672 816

## Description

L'invention se rapporte à un dispositif notamment destiné à l'hygiène des conduits auditifs de personnes.

L'invention concerne spécialement un dispositif qui, du type précité, comprend un corps longiligne de forme sensiblement cylindrique présentant deux extrémités dont l'une au moins porte, généralement, sans solution de continuité une partie active creuse destinée à recueillir un matériau tel le cerumen.

Sur les dispositifs de l'art antérieur tels ceux décrits dans les documents (US-A-1.672.816, FR-A-2.277.557, FR-A-2.551.657), la partie active précitée est constituée par une sorte de spatule arrondie qui présente une légère dépression.

Les parties actives connues sont donc notablement plates et constituent donc une sorte de cuillère. FR-A-2 551 657 décrit un dispositif dit d'hygiène auriculaire selon le préambule de la revendication 1.

Les dispositifs ainsi équipés ont leur efficacité mais on déplore que leur utilisation soit relativement dangereuse pour le tympan.

Pour remédier à cet inconvénient, sans altérer l'efficacité du dispositif, il est connu (FR-A-2.277.557) d'équiper le corps du dit dispositif d'une butée disposée et orientée pour, lors de l'utilisation, venir coopérer avec un épaulement naturel du conduit auditif et, par cela, limiter l'engagement de la partie active dans le conduit.

Cet aménagement est efficace mais augmente notablement le coût de fabrication du dispositif.

Le résultat essentiel que l'invention vise à obtenir est un dispositif d'hygiène auriculaire qui, tout en étant efficace et sans danger, est de moindre coût que ceux connus.

A cet effet, l'invention a pour objet un dispositif d'hygiène auriculaire notamment caractérisé en ce que chaque partie active a une forme d'enveloppe demi-sphérique de rayon déterminé.

L'invention sera bien comprise à l'aide de la description ci-après faite à titre d'exemple non limitatif en regard du dessin ci-annexé qui représente schématiquement :

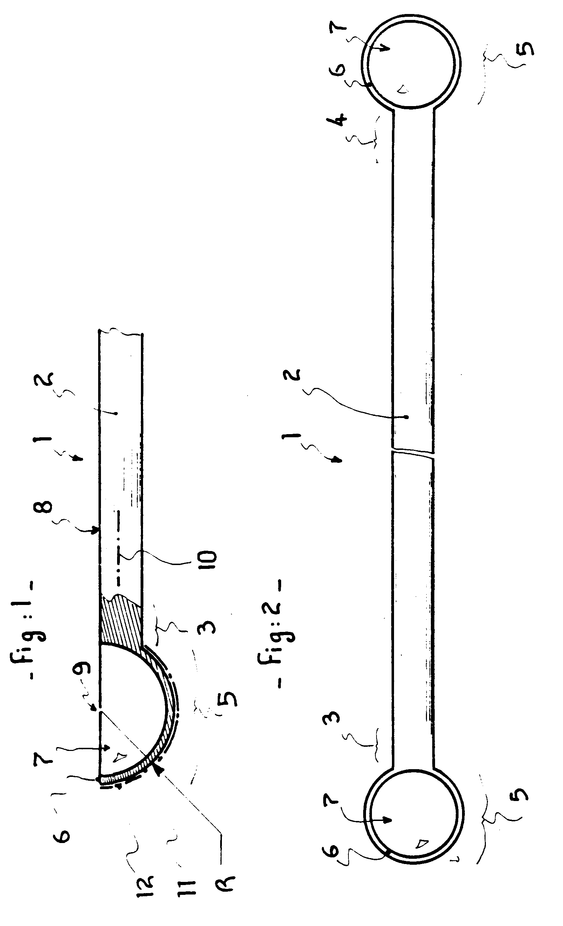

- figure 1 : a grande échelle, une vue en coupe transversale de la partie active du dispositif,
- figure 2 : à grande échelle en vue de face complète, le dispositif selon l'invention.

En se reportant au dessin, on voit un dispositif 1 dit d'hygiène auriculaire parce que notamment destiné à l'hygiène des conduits auditifs de personnes (non représentées).

Classiquement, le dispositif comprend un corps cylindrique 2 présentant deux extrémités 3, 4 opposées dont l'une au moins porte, notamment sans solution de continuité, une partie active 5 qui, elle-même, comporte, d'une part, un bord actif 6 sensiblement circulaire destiné à détacher un matériau d'une surface déterminée et, d'autre part, une dépression ou cavité 7 destinée à recueillir le dit matériau.

Comme cela est représenté et de manière caractéristique pour l'invention, chaque partie active 5 a une forme d'enveloppe demi-sphérique de rayon R déterminé.

Selon l'invention, le bord 6 sensiblement circulaire de la partie active 5 est situé dans un plan approximativement tangent à l'une 8 des génératrices externes du corps cylindrique 2 qui porte cette partie active 5.

De préférence, la cavité 7 que comporte la partie active demi-sphérique est elle-même demi-sphérique.

Conformément à l'invention, le point 9 d'origine de tout rayon de la partie active 5 est situé approximativement dans un plan qui contient l'axe longitudinal 10 du corps 2.

Ainsi constitué et pour peu que le rayon de la partie active demi-sphérique soit choisi de manière à limiter l'engagement dans le conduit auditif à une valeur prédéterminée.

Le dispositif selon l'invention ne peut engendrer de lésion puisque le bord 6 circulaire de la partie active ne peut être exploité pour prélever le cerumen que par des déplacements tangentiels à la face externe de cette partie active.

Selon l'invention, au moins une fraction de la surface externe 11 de la partie active 5 porte un revêtement 12 souple présentant de fines aspérités en vue de retenir tout matériau ou corps gras au contact duquel il se trouve placé.

Conformément à l'invention, lorsque le dispositif est destiné à l'usage d'un enfant, le rayon de la partie demi-sphérique est supérieur à 2,25 millimètres.

Suivant l'invention, lorsque le dispositif est destiné à l'usage d'un adulte, le rayon de la partie demi-sphérique est supérieur à 3,35 millimètres.

De préférence, d'une part, le corps 2 du dispositif 1 et sa partie active 5 sont constitués dans un matériau résilient et, d'autre part, le corps 2 présente une certaine flexibilité.

Ces deux derniers paramètres de construction sont à la portée de l'homme de l'art.

## Revendications

1. Dispositif dit d'hygiène auriculaire car notamment destiné à l'hygiène des conduits auditifs de personnes, lequel dispositif comprend un corps cylindrique (2) présentant deux extrémités (3, 4) opposées dont l'une au moins porte, notamment sans solution de continuité, une partie active (5) qui, elle-même, comporte, d'une part, un bord actif (6) sensiblement circulaire destiné à détacher un matériau d'une surface déterminée et, d'autre part, une dépression ou cavité (7) destinée à recueillir le dit matériau,
ce dispositif étant **CARACTERISE** en ce que chaque partie active (5) a une forme d'enveloppe demi-sphérique de rayon (R) déterminé.

2. Dispositif selon la revendication 1 **caractérisé** en ce que le bord (6) sensiblement circulaire de la partie active (5) est situé dans un plan approximativement tangent à l'une (8) des génératrices externes du corps cylindrique (2) qui porte cette partie active (5).

3. Dispositif selon la revendication 1 ou 2 **caractérisé** en ce que le point (9) d'origine de tout rayon de la partie active (5) est situé approximativement dans un plan qui contient l'axe longitudinal (10) du corps (2).

4. Dispositif selon l'une quelconque des revendications 1 à 3 **caractérisé** en ce qu'au moins une fraction de la surface externe (11) de la partie active (5) porte un revêtement (12) souple présentant de fines aspérités en vue de retenir tout matériau ou corps gras au contact duquel il se trouve placé.

5. Dispositif selon l'une quelconque des revendications 1 à 4 **caractérisé** en ce que, lorsque le dispositif est destiné à l'usage d'un enfant, le rayon de la partie demi-sphérique est supérieur à 2,25 millimètres.

6. Dispositif selon l'une quelconque des revendications 1 à 4 **caractérisé** en ce que, lorsque le dispositif est destiné à l'usage d'un adulte, le rayon de la partie demi-sphérique est supérieur à 3,35 millimètres.

## Claims

1. A device known as an auricular hygiene device because it is intended in particular for the hygiene of the auditory canals in people, the said device comprising a cylindrical body (2) having two opposing ends (3, 4), at least one of which carries, in particular without a gap, an active part (5) which itself comprises firstly a substantially circular active edge (6) for removing matter from a predetermined surface, and secondly a depression or cavity (7) for collecting the said matter, this device being characterised in that each active part (5) is in the form of a hemi-spherical casing of a specific radius (R).

2. A device according to claim 1, characterised in that the substantially circular edge (6) of the active part (5) is located in a plane approximately tangential to one (8) of the external generatrices of the cylindrical body (2) carrying this active part (5).

3. A device according to claim 1 or 2, characterised in that the point (9) of origin of any radius of the active part (5) is approximately located in a plane containing the longitudinal axis (10) of the body (2).

4. A device according to any one of claims 1 to 3, characterised in that at least one portion of the external surface (11) of the active part (5) is provided with a flexible coating (12) having fine asperities in order to retain any greasy substances or matter with which it is placed in contact.

5. A device according to any one of claims 1 to 4, characterised in that the radius of the hemi-spherical part is greater than 2.25 millimetres when the device is intended for use in a child.

6. A device according to any one of claims 1 to 4, characterised in that the radius of the hemi-spherical part is greater than 3.35 millimetres when the device is intended for use in an adult.

## Patentansprüche

1. Reinigungsgerät, insbesondere Ohrreinigungsgerät, das zur Reinigung der Gehörgänge beim Menschen bestimmt ist, weiches ein zylinderförmiges Hauptteil (2) mit zwei gegenüberliegenden Enden (3, 4) aufweist, von denen mindestens eines direkt anschließend einen Wirkbereich (5) aufweist, der seinerseits sowohl eine im wesentlichen kreisförmige Wirkkante (6) zum Ablösen von Material von einer bestimmten Fläche als auch eine zum Auffangen des abgelösten Materials bestimmte Vertiefung bzw. einen Hohlraum (7) trägt,
**dadurch GEKENNZEICHNET**, daß jeder Wirkbereich (5) eine halbkugelförmige Außenform mit vorgegebenem Radius (R) aufweist.

2. Reinigungsgerät nach Anspruch 1, **dadurch gekennzeichnet,** daß die im wesentlichen kreisförmige Wirkkante (6) des Wirkbereichs (5) in einer Ebene liegt, die in etwa tangential zu einer (8) der äußeren Erzeugenden des zylinderförmigen Hauptteils (2) verläuft, welches diesen Wirkbereich (5) trägt.

3. Reinigungsgerät nach Anspruch 1 oder 2, **dadurch gekennzeichnet**, daß der Ursprung (9) jedes Radius des Wirkbereichs (5) in etwa in einer Ebene liegt, in welcher die Längsachse (10) des Hauptteils (2) verläuft.

4. Reinigungsgerät nach einem der Ansprüche 1 bis 3, **dadurch gekennzeichnet,** daß zumindest ein Teil der Außenfläche (11) des Wirkbereichs (5) eine elastische Beschichtung (12) aufweist, die feine Unebenheiten besitzt, um jegliches Material bzw. alle Fetteilchen festzuhalten, mit denen sie in Kontakt kommt.

5. Reinigungsgerät nach einem der Ansprüche 1 bis 4, **dadurch gekennzeichnet,** daß in den Fällen, in denen das Gerät zur Verwendung bei Kindern vorgesehen ist, der Radius des halbkugelförmigen Abschnitts länger als 2,25 mm ist.

6. Reinigungsgerät nach einem der Ansprüche 1 bis 4, **dadurch gekennzeichnet,** daß in den Fällen, in denen das Gerät zur Verwendung bei Erwachsenen vorgesehen ist, der Radius des halbkugelförmigen Abschnitts länger als 3,35 mm ist.
